# EUROPÄISCHE PATENTSCHRIFT

(11) **EP 3 037 109 B1**
(45) Veröffentlichungstag und Bekanntmachung des Hinweises auf die Patenterteilung: **13.09.2017**
(21) Anmeldenummer: 15201909.7
(22) Anmeldetag: 22.12.2015
(51) Int. Cl.: A61L 2/18, B08B 9/42

(54) **BEHÄLTERZELLENTRÄGER ZUM TRAGEN MINDESTENS EINER BEHÄLTERZELLE UND REINIGUNGSMASCHINE**
CONTAINER CELL CARRIER FOR CARRYING AT LEAST ONE CONTAINER CELL AND CLEANING MACHINE
SUPPORT DE CELLULE DE RECIPIENT DESTINE A PORTER AU MOINS UNE CELLULE DE RECIPIENT ET MACHINE DE NETTOYAGE

(30) Priorität: 22.12.2014 DE 102014119433
(43) Veröffentlichungstag der Anmeldung: 29.06.2016
(73) Patentinhaber: KRONES AG, 93073 Neutraubling (DE)
(72) Erfinder: Sell, David, 93073 Neutraubling (DE); Soerensen, Matthias, 93073 Neutraubling (DE)
(74) Vertreter: Nordmeyer, Philipp Werner

(56) Entgegenhaltungen:
- EP-A2- 1 281 447
- DE-B- 1 147 862
- DE-U1- 20 112 612
- GB-A- 689 729

## Beschreibung

### Technisches Gebiet

Die vorliegende Erfindung betrifft einen Behälterzellenträger zum Tragen mindestens einer Behälterzelle zur Aufnahme eines zu reinigenden Behälters in einer Reinigungsmaschine, bevorzugt in einer Reinigungsmaschine einer Getränkeabfüllanlage, und eine einen solchen Behälterzellenträger aufweisende Reinigungsmaschine zum Reinigen von Behältern.

### Stand der Technik

Behälterzellenträger zum Tragen von Behälterzellen in Reinigungsmaschinen sind wohlbekannt. Hierbei gewährleisten die durch den Behälterzellenträger gehaltenen Behälterzellen während des Reinigungsprozesses einen schonenden Transport der zu reinigenden Behälter durch die Reinigungsmaschine. Die zu reinigenden Behälter werden dabei mittels einer Einführvorrichtung in die Behälterzellen eingeführt und anschließend durch die Bewegung des Behälterzellenträgers durch die Reinigungsmaschine geführt. Dabei durchläuft der Behälterzellenträger während der Reinigung verschiedene Behandlungszonen der Reinigungsmaschine, in welchen unterschiedliche Reinigungsmedien bei unterschiedlichen Temperaturen vorliegen.

Entsprechend der spezifischen Wärmekapazität des oder der Behälterzellenwerkstoffe und der Masse des Behälterzellenträgers, sowie der in einer Behandlungszone vorliegenden Temperatur und der Aufenthaltsdauer des Behälterzellenträgers in der Behandlungszone nimmt der Behälterzellenträger eine gewisse Menge an Wärmeenergie auf. Bei einem Wechsel des Behälterzellenträgers in eine weitere Behandlungszone mit geringerer Temperatur verschleppt der Behälterzellenträger diese Wärmemenge in diese weitere Behandlungszone. Dies kann teilweise gewollt sein, beispielsweise wenn ein Erwärmen der weiteren Behandlungszone durch die Abwärme des Behälterzellenträgers vorgesehen ist. Jedoch kann die Abgabe der aufgenommenen Wärmeenergie auch dazu führen, dass die weitere Behandlungszone beziehungsweise deren Komponenten zu stark erwärmt werden und folglich prozesstechnisch gegenzusteuern ist, beispielsweise durch Vorsehen einer zusätzlichen Kühlung oder eine Abkühlzone zwischen den beiden Behandlungszonen. Dies wiederum führt zu einem erhöhten Energiebedarf zum Betrieb der Reinigungsmaschine, da unter anderem durch die Kühlung zusätzlich Energie aufzuwenden ist und aufgrund der stetigen Wärmeabfuhr durch die Behälterzellenträger aus der Behandlungszone mit hoher Temperatur ein hoher Heizenergiebedarf zum Kompensation der Wärmeabfuhr entsteht.

Aufgrund des hohen Eigengewichts des Behälterzellenträgers wird somit eine unnötig hohe Menge an Wärmeenergie in den Behandlungszonen der Reinigungsmaschine verschleppt. Ferner sind die in der Reinigungsmaschine verbauten Antriebe zur Bewegung der Behälterzellenträger aufgrund deren hohen Eigengewichts entsprechend groß auszulegen und folglich energieintensiv zu betreiben.

Aus dem Stand der Technik sind Behälterzellenträger bekannt, welche ein reduziertes Eigengewicht aufweisen. So zeigt die EP 1 281 447 A2 einen Flaschenzellenträger eines Flaschenkorbes für Reinigungsmaschinen, welcher aus einem U-förmig abgewinkelten Profilträger mit zwei Schenkeln und einem mit den Schenkeln verbundenen Jochabschnitt besteht. Hierbei sind die Schenkel mit großformatigen Öffnungen zur Erzeugung einer gitter- oder leiterartigen Struktur versehen. Zwar kann durch diese Ausgestaltung ein reduziertes Eigengewicht und somit eine reduzierte Wärmeenergieaufnahme und -abgabe bereitgestellt werden. Jedoch weist der Flaschenzellenträger durch die leiterartige Struktur reduzierte Biegesteifigkeiten, Verwindungssteifigkeiten und Schwingungssteifigkeiten auf. Die verminderte Stabilität der Flaschenzellenträger beziehungsweise Behälterzellenträger in dieser Form kann wiederum negative Auswirkungen auf Komponenten der Reinigungsmaschine haben, etwa kann die Lagerung der Behälterzellenträger durch auftretende Schwingungen der Behälterzellenträger stärker belastet werden, und/oder negative Auswirkungen auf die in den Behälterzellen befindlichen Behälter selbst haben, welche aufgrund der reduzierten Stabilität der Behälterzellenträger beschädigt werden oder unzureichend gereinigt werden könnten. Ferner wird durch den im Wesentlichen horizontal ausgerichteten Jochabschnitt auch stets eine gewisse Menge an Flüssigkeit von einer Behandlungszone in die folgende oder die folgenden Behandlungszonen transportiert.

### Darstellung der Erfindung

Ausgehend von dem bekannten Stand der Technik ist es eine Aufgabe der vorliegenden Erfindung, einen verbesserten Behälterzellenträger zum Tragen mindestens einer Behälterzelle zur Aufnahme eines zu reinigenden Behälters in einer Reinigungsmaschine bereitzustellen.

Diese Aufgabe wird mit einem Behälterzellenträger zum Tragen mindestens einer Behälterzelle zur Aufnahme eines zu reinigenden Behälters in einer Reinigungsmaschine mit den Merkmalen des Anspruch 1 gelöst. Vorteilhafte Weiterbildungen ergeben sich aus den Unteransprüchen.

Entsprechend wird ein Behälterzellenträger zum Tragen mindestens einer Behälterzelle zur Aufnahme eines zu reinigenden Behälters in einer Reinigungsmaschine, bevorzugt in einer Reinigungsmaschine einer Getränkeabfüllanlage, vorgeschlagen, welche mindestens eine sich in einer Längsachse erstreckende Tragleiste zum Halten der mindestens einen Behälterzelle zum Transport durch die Reinigungsmaschine umfasst. Erfindungsgemäß weist die mindestens eine Tragleiste einen Fachwerkbereich mit in Richtung der Längsachse regelmäßig angeordneten Aussparungen zum Ausbilden einer fachwerkartigen Struktur auf, wobei durch die Aussparungen in der Tragleiste Stabsegmente und Knotenpunkte bereitgestellt sind und wobei sich jeweils mindestens zwei der Stabsegmente in jeweils einem Knotenpunkt des Fachwerkbereichs treffen.

Dadurch, dass die mindestens eine Tragleiste einen Fachwerkbereich mit in Richtung der Längsachse regelmäßig angeordneten Aussparungen zum Ausbilden einer fachwerkartigen Struktur aufweist, wobei durch die Aussparungen in der Tragleiste Stabsegmente und Knotenpunkte bereitgestellt sind und wobei sich jeweils mindestens zwei der Stabsegmente in jeweils einem Knotenpunkt des Fachwerkbereichs treffen, kann eine stabile Struktur des Behälterzellenträgers in Bezug auf sein Eigengewicht mit hohen Steifigkeiten und somit mit einer hohen Stabilität bereitgestellt werden. Durch die Verbindung der mindestens zwei Stabsegmente in einem Knotenpunkt können die im Betrieb der Reinigungsmaschine auf den Behälterzellenträger wirkenden Kräfte effektiv aufgenommen und weitergeleitet werden. Der Kraftfluss verläuft hierbei ausgehend von den Knotenpunkten über die Stabsegmente, so dass die Bereiche, in welchen die Aussparungen angeordnet sind, unbelastete Bereiche der mindestens einen Tragleiste sind. Folglich können diese Bereiche ausgespart werden, ohne die Stabilität des Behälterzellenträgers stark zu verringern. Weiterhin kann aufgrund des im Vergleich zu einer Tragleiste aus Vollmaterial reduziertem Gewichts der Tragleiste die Durchbiegung des Behälterzellenträgers aufgrund seines Eigengewichts verringert werden.

Ferner ist aufgrund der verringerten Masse des Behälterzellenträgers die durch den Behälterzellenträger aufgenommene und in anschießende Behandlungszonen verschleppte Wärmemenge im Vergleich zu einem Behälterzellenträger mit einer Tragleiste aus Vollmaterial reduziert.

Auch können die Antriebe der Reinigungsmaschine aufgrund des verringerten Gewichts des Behälterzellenträgers kleiner ausgelegt werden, was den Energieverbrauch der Reinigungsmaschine weiter senkt. Gleichzeitig erwachsen aus dem Einsatz des Behälterzellenträgers in dieser Form keine erhöhten Anforderungen an die Lagerung der Behälterzellenträger und die Transportkette der Reinigungsmaschine, da die Behälterzellenträger keine oder eine lediglich geringe Reduzierung der Stabilität des Behälterzellenträgers in Vergleich zu einem Behälterzellenträger aus Vollmaterial aufweist.

In einer bevorzugten Weiterbildung weist der Behälterzellenträger zwei Tragleisten auf, die in Richtung der Längsachse gesehen beidseitig der mindestens einen Behälterzelle angeordnet sind. Bevorzugt sind die Tragleisten spiegelsymmetrisch bezüglich einer Ebene, welche aus der Behälterzellenmittelachse der mindestens einen Behälterzelle und der die Behälterzellenmittelachse senkrecht schneidende Längsachse des Behälterzellenträgers gebildet wird, angeordnet.

Dadurch kann eine besonders stabile Struktur des Behälterzellenträgers in Bezug auf sein Eigengewicht mit besonders hohen Steifigkeiten und somit einer besonders hohen Stabilität bereitgestellt werden.

Bevorzugt weist der Behälterzellenträger eine Mehrzahl an Behälterzellen auf. Dadurch kann eine besonders effektive Reinigungsmaschine bereitgestellt werden.

Erfindungsgemäß ist mindestens ein Knotenpunkt des Fachwerkbereichs in Richtung der Längsachse gesehen auf Höhe einer senkrecht zur Längsachse orientierten Behälterzellenmittelachse mindestens einer Behälterzelle angeordnet. Bevorzugt ist jeweils genau ein Knotenpunkt des Fachwerkbereichs in Richtung der Längsachse gesehen auf Höhe der Behälterzellenmittelachse jeweils einer Behälterzelle angeordnet.

Dadurch, dass mindestens ein Knotenpunkt des Fachwerkbereichs in Richtung der Längsachse gesehen auf Höhe einer senkrecht zur Längsachse orientierten Behälterzellenmittelachse mindestens einer Behälterzelle angeordnet ist, kann eine besonders stabile Struktur des Behälterzellenträgers bereitgestellt werden. Die Projektion des Kraftvektors einer durch die einen Behälter aufgenommene Behälterzelle in die mindestens eine Tragleiste eingeleiteten Kraft liegt hierdurch in dem Knotenpunkt, sodass keine oder in nur sehr geringem Maße Momente auf den Knotenpunkt wirken und die Stabsegmente lediglich Zug- und Druckkräfte übertragen. Folglich ist in dieser Weiterbildung die Ausbildung eines quasiidealen Fachwerkbereichs, in welchem nur entlang einer Stabsegmentlängsachse der Stabsegmente Kräfte übertragen werden und Kräfte nur in den Knotenpunkten angreifen, möglich.

In einer besonders bevorzugten Ausführungsform schließen die Stabsegmente mit ihrer Stabsegmentlängsachse mit der Längsachse der mindestens einen Tragleiste einen Winkel, bevorzugt in einem Winkelbereich von größer 0° und kleiner 90°, ein.

Dadurch, dass die Stabsegmente mit ihrer Stabsegmentlängsachse mit der Längsachse der mindestens einen Tragleiste einen Winkel, bevorzugt in einem Winkelbereich von größer 0° und kleiner 90°, einschließen, kann eine bezogen auf ihr Eigengewicht besonders stabile Struktur bereitgestellt werden, da die angewinkelten Stabsegmente sowohl Kräfte in Richtung der Längsachse, sowie Kräfte quer zu dieser übertragen können. Die Stabsegmente können Zug- und Druckkräfte in Richtung ihrer Stabsegmentlängsachse übertragen. Da die Stabsegmentlängsachse mit der Längsachse einen Winkel einschließt, kann eine in Stabsegmentlängsachse gerichtete Kraft entsprechend des eingeschlossenen Winkels in eine Komponente in Längsrichtung und in eine Komponente quer dazu zerlegt werden, welche beide in ihren Betrag ungleich null sind. Somit kann der Fachwerkbereich als quasiideales Fachwerk bereitgestellt werden, bei welchem die äußeren und inneren Kräfte durch die Knotenpunkte direkt über die Stabsegmente übertragen werden, ohne dass in die Stabsegmente starke Momente eingeleitet werden, wie dies bei einer Tragleiste mit leiterartiger Struktur der Fall ist. Dementsprechend kann ein Behälterzellenträger mit solch einer Struktur in Vergleich zu einem Behälterzellenträger mit mindestens einer Tragleiste aus Vollmaterial leichtbauend, folglich mit geringem Gewicht, bereitgestellt werden, ohne die Stabilität des Behälterzellenträgers signifikant zu beeinträchtigen. Weiterhin kann ein Behälterzellenträger mit angewinkelten Tragleisten im Vergleich zu einem Behälterzellenträger mit gitter- oder leiterartiger Struktur bei ähnlichem Eigengewicht besonders stabil sowie biege- und verdrehfest bereitgestellt werden.

In einer bevorzugten alternativen Weiterbildung beträgt die Gesamtfläche der Aussparungen höchstens 50 % der Gesamtfläche der Tragleiste.

Hierdurch kann bereitgestellt werden, dass der Behälterzellenträger seine für den Betrieb in der Reinigungsmaschine erforderliche Stabilität und seine erforderlichen Festigkeiten aufweist und weiterhin die durch den Behälterzellenträger verschleppte Wärmemenge gering gehalten ist.

In einer weiter bevorzugten Ausführungsform sind die Behälterzellen mit einem Abstand zwischen den Behälterzellenmittelachsen zweier benachbarter Behälterzellen angeordnet, wobei die Behälterzellen eine Behälterzellenbreite aufweisen und wobei der Abstand bevorzugt der Behälterzellenbreite entspricht.

Dadurch, dass die Behälterzellen mit einem Abstand angeordnet sind, kann eine regelmäßige Struktur des Behälterzellenträgers bereitgestellt werden, wodurch eine gleichmäßige Verteilung der auf den Behälterzellenträger wirkenden Kräfte und der von dem Behälterzellenträger auf seine Lagerung und seine Anbindung in der Reinigungsmaschine übertragenen Kräfte bereitgestellt werden. Entspricht der Abstand der Behälterzellenbreite, so können die Behälterzellen direkt aneinander befestigt beziehungsweise direkt voneinander gehalten werden, mit anderen Worten Behälterzelle an Behälterzelle, was die Stabilität des Behälterzellenträgers weiter erhöht, ohne dass zusätzliche Teile notwendig sind. Eine solche direkte Verbindung der Behälterzellen miteinander ist besonders bevorzugt bei einer Ausgestaltung der Behälterzellen aus Metall vorzusehen.

In einer besonders bevorzugten Ausgestaltung ist mindestens eine Aussparung als Dreieck bereitgestellt, bevorzugt sind alle Aussparungen als Dreiecke bereitgestellt.

Dadurch kann erreicht werden, dass die durch die mindestens eine Aussparung in Form eines Dreiecks gebildeten Stabsegmente derart angeordnet sind, dass Kräfte in mindestens zwei Richtungen übertragen werden können.

In einer bevorzugten Ausführungsform ist mindestens eine Ecke mindestens einer Aussparung abgerundet, bevorzugt sind alle Ecken der Aussparung, besonders bevorzugt alle Ecken aller Aussparungen, abgerundet bereitgestellt.

Dadurch kann ein verbesserter Kraftfluss mit einer verringerten Kerbwirkung in der oder den Ecken erreicht werden, was die Stabilität und die Dauerfestigkeit des Behälterzellenträgers weiterhin verbessert. Ferner kann der Behälterzellenträger hygienisch verbessert bereitgestellt werden, da sich in Kanten und Ecken Schmutz und anderweitige Beläge ablagern beziehungsweise festsetzen können. Durch das Vorsehen der Rundung können diese Ecken und Kanten vermieden werden.

In einer bevorzugten weiteren Ausführungsvariante ist in dem Fachwerkbereich mindestens ein Verbindungssegment zum Versteifen des Fachwerkbereichs zwischen den Stabsegmenten, bevorzugt parallel zur Längsachse orientiert, angeordnet und/oder zwischen den Tragleisten senkrecht zur Längsachse mindestens eine Schottwand zum Versteifen des Behälterzellenträgers angeordnet, bevorzugt wenn die mindestens eine Behälterzelle Kunststoff aufweist beziehungsweise die Behälterzelle aus Kunststoff gefertigt ist. Solche Behälterzellen aus Kunststoff werden in den Behälterzellenträger bevorzugt über einen Formschluss, beispielsweise einen Bajonettverschluss, verriegelbar eingesetzt.

Durch das mindestens eine Verbindungssegment kann unter anderem die Struktur des Fachwerkbereichs weiter versteift werden. Insbesondere können hierdurch die Stabsegmente gegen Knicken versteift werden. Auch kann durch das Vorsehen mehrerer Verbindungsegmente, bevorzugt parallel zur Längsachse, ein im Fachwerkbereich durchgehender Materialanschnitt bereitgestellt werden, an welchem beispielsweise bei einer Ausgestaltung der Tragleiste als Biegeblech ein durchgehender Biegefalz angeordnet sein kann, was die Stabilität des Behälterzellenträgers weiter erhöht und zusätzlich die Fertigung des Behälterzellenträgers erleichtert. Durch das Vorsehen der Schottwand kann der Behälterzellenträger weiter stabilisiert werden. Weiterhin kann die Schottwand einstückig mit der mindestens einen Tragleiste verbunden sein, bevorzugt sind diese als ein Teil in einem Fertigungsschritt hergestellt. Alternativ können auch mehrere Fertigungsschritte sowie Materialkombinationen verwendet werden.

Das mindestens eine Verbindungssegment ist hierbei ferner bevorzugt als Restblech beziehungsweise Restblechbereich bereitgestellt, welcher als einstückig mit der mindestens einen Tragleiste verbundener Materialbereich in den Bereich der das mindestens eine Verbindungssegment aufweisende Aussparung verbleibt. Das mindestens eine Verbindungssegment, teilt die Aussparungen hierbei bevorzugt in mindestens ein Aussparungsdreieck und mindestens ein Aussparungstrapez. Durch den mittels des Verbindungssegments somit bereitgestellten durchgehenden Materialabschnitt kann die Tragleiste zusätzlich stabilisiert werden. Alternativ kann das mindestens eine Verbindungssegment die Aussparung auch in Teilaussparungen anderer Form teilen.

In einer besonders bevorzugten Ausführungsvariante ist die mindestens eine Tragleiste als Profilleiste bereitgestellt.

Durch die Bereitstellung der mindestens einen Tragleiste als Profilleiste kann eine besonders hohe Stabilität, insbesondere gegen Biegung, bereitgestellt werden. Weiterhin weist der Behälterzellenträger hierdurch eine erhöhte Schwingungsfestigkeit auf. Die Profilleiste weist dabei in einem Schnitt senkrecht zur Längsrichtung einen Querschnitt auf, welcher zweidimensional ausprägt ist. Mit anderen Worten ist die Ausprägung des Querschnitts in die beiden orthogonal zueinander orientierten Dimensionen des Querschnitts jeweils größer, als die Wandstärke der Profilleiste.

In einer bevorzugten alternativen Ausbildung ist mindestens ein Verbindungsbereich zum Verbinden mindestens zweier Tragleisten bereitgestellt, wobei die Tragleisten beidseitig an dem Verbindungsbereich angeordnet sind und wobei die Tragleisten und der Verbindungsbereich bevorzugt als einstückiges U-Profil bereitgestellt sind, wobei der Verbindungsbereich bevorzugt seitlich, oberhalb und/oder unterhalb der Tragleisten angeordnet ist.

Hierdurch kann der Behälterzellenträger zusätzlich stabilisiert werden. Ferner kann der Verbindungsbereich zur Herstellung des Behälterellenträgers aus einem Stück dienen. So kann ein stirnseitig angeordneter Verbindungsbereich als Anguss für zwei Tragleisten dienen.

In einer bevorzugten Weiterbildung ist mindestens eine Anbindungsanordnung zum Anbinden an eine Transportkette der Reinigungsmaschine, bevorzugt stirnseitig, angeordnet.

Dadurch, dass mindestens eine Anbindungsanordnung zum Anbinden an eine Transportkette der Reinigungsmaschine, bevorzugt stirnseitig, angeordnet ist, kann eine einfache, sichere und dauerhafte Anbindung an eine Transportkette der Reinigungsmaschine mit einer sicheren Kraftübertragung bereitgestellt werden. Ferner kann die Anbindungsanordnung, insbesondere dann, wenn sie stirnseitig angeordnet ist, zur zusätzlichen Versteifung des Behälterzellenträgers beitragen.

In einer bevorzugten Ausführungsvariante ist die mindestens eine Behälterzelle eingesteckt.

Hierdurch kann der Behälterzellenträger besonders einfach und kostengünstig gefertigt werden. Bevorzugt ist an dem Behälterzellenträger dabei mindestens ein Halteelement zum Halten und/oder Positionieren der mindestens einen Behälterzelle vorgesehen, besonders bevorzugt in Form einer Haltelasche.

In einer bevorzugten Weiterführung ist die mindestens eine Behälterzelle mittels mindestens eines Fügebereichs, bevorzugt mittels Kleben, Schweißen, Verschrauben, Nieten und/oder Schmelzen, an der mindestens einen Tragleiste gehalten.

Durch das Vorsehen des mindestens Fügebereichs kann die mindestens eine Behälterzelle dauerhaft in ihrer Position gesichert werden. Weiterhin kann durch den mindestens einen Fügebereich eine zusätzliche Versteifung des Behälterzellenträgers bereitgestellt werden. Der mindestens eine Fügebereich kann dabei punktförmig, linienförmig oder flächig ausgebildet sein. Im Falle, dass an dem Behälterzellenträger mehrere Behälterzellen vorgesehen sind, kann auch mindestens ein weiterer Fügebereich zwischen den Behälterzellen und oder zwischen mindestens einer Behälterzelle und mindestens einer Schottwand und/oder mindestens einer Anbindungsanordnung vorgesehen sein.

In einer bevorzugten Weiterbildung sind die Aussparungen, durch Heraustrennen, bevorzugt Stanzen und/oder ein Beschnittverfahren wie Laserstrahlschneiden oder Wasserstrahlschneiden, aus einem Vollmaterial der mindestens einen Tragleiste bereitgestellt, wobei die mindestens eine Tragleiste aus einem Kunststoff und/oder aus einem Metall oder einer Metalllegierung, bevorzugt Stahl, besonders bevorzugt als Biegeblech, Stanzblech, Spritzgussteil und/oder Extrusionsprofil, bereitgestellt ist.

Dadurch kann ein besonders kostengünstig und einfach gefertigter Behälterzellenträger mit einer hohen Stabilität und einem geringen Gewicht bereitgestellt werden. Ferner kann der Behälterzellenträger hierdurch für den Einsatz im Hygienebereich geeignet ausgebildet sein, da die Materialien resistent gegen Reinigungsmittel sein können, wodurch auch ein dauerhafter Einsatz des Behälterzellenträgers in der Reinigungsmaschine mit großen Wartungsintervallen bereitgestellt werden kann. Ferner kann der Behälterzellenträger durch den Einsatz großserientauglicher Fertigungsschritte hergestellt sein, was die Fertigungskosten des Behälterzellenträgers gering hält.

Die oben genannte zu lösende Aufgabe wird auch durch eine Reinigungsmaschine mit den Merkmalen des unabhängigen Anspruchs 15 gelöst.

Entsprechend wird eine Reinigungsmaschine zum Reinigen von Behältern angegeben, welche eine Transportkette mit einer Transportrichtung und mindestens eine Reinigungseinrichtung zum Reinigen der Behälter umfasst. Erfindungsgemäß ist eine Vielzahl an Behälterzellenträgern gemäß einer der vorstehenden Ausführungsformen entlang der Transportkette der Reinigungsmaschine angeordnet, wobei die Behälterzellenträger hinsichtlich ihrer Längsachse quer zu der Transportrichtung angeordnet sind.

Dadurch, dass eine Vielzahl an Behälterzellenträgern gemäß einer der vorstehenden Ausführungsformen entlang der Transportkette der Reinigungsmaschine angeordnet ist, wobei die Behälterzellenträger hinsichtlich ihrer Längsachse quer zu der Transportrichtung angeordnet sind, kann eine Reinigungsmaschine mit geringem Energiebedarf bereitgestellt werden, da der Energiebedarf für das Heizen, das Kühlen und/oder der Energiebedarf der Antriebe im Vergleich zu einer mit herkömmlichen Behälterzellenträgern ausgestatteten Reinigungsmaschine reduziert ist.

### Kurze Beschreibung der Figuren

Bevorzugte weitere Ausführungsformen der Erfindung werden durch die nachfolgende Beschreibung der Figuren näher erläutert. Dabei zeigen:
- Figur 1: schematisch einen Behälterzellenträger mit einer Mehrzahl an Behälterzellen zur Aufnahme eines zu reinigenden Behälters in einer Reinigungsmaschine in einer perspektivischen Seitenansicht;
- Figur 2: schematisch eine Tragleiste des Behälterzellenträgers aus Figur 1 in einer Seitenansicht;
- Figur 3: schematisch einen Querschnitt durch die Trageleiste des Behälterzellenträgers aus Figur 2 senkrecht zur Längsachse in einem Vollmaterialbereich;
- Figur 4: schematisch einen Querschnitt durch die Trageleiste des Behälterzellenträgers aus Figur 2 senkrecht zur Längsachse in einem Fachwerkbereich;
- Figur 5: schematisch eine Seitenansicht des Behälterzellenträgers 1 aus Figur 1, und
- Figur 6: schematisch eine perspektivische Teilansicht eines Behälterzellenträgers in einer alternativen Ausführungsform.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet. Um Redundanzen zu vermeiden, wird auf die wiederholte Beschreibung dieser Elemente in der nachfolgenden Beschreibung teilweise verzichtet.

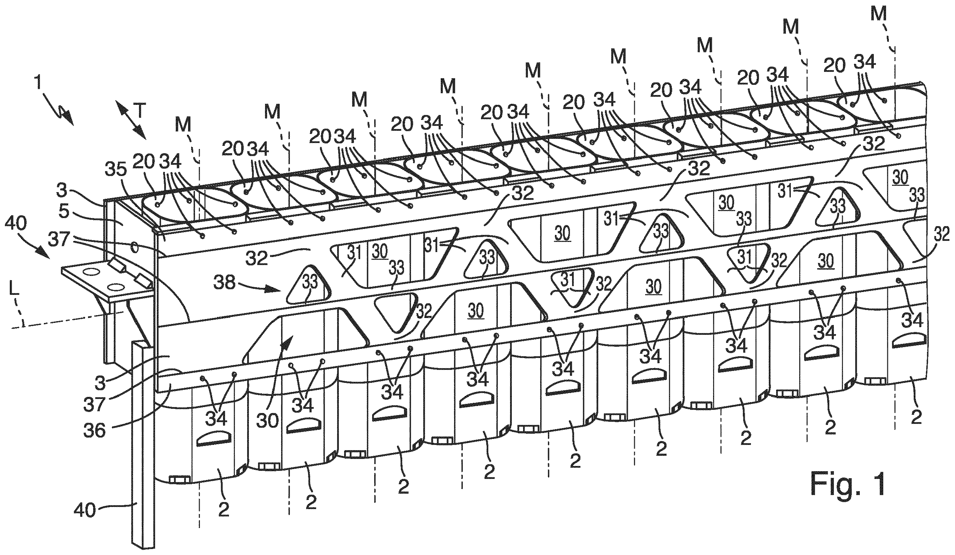

Figur 1 zeigt schematisch einen Behälterzellenträger 1 mit einer Mehrzahl an Behälterzellen 2 zur Aufnahme eines zu reinigenden Behälters in einer Reinigungsmaschine in einer perspektivischen Seitenansicht. Der Behälterzellenträger weist beidseitig einer Längsachse L angeordnete Tragleisten 3 auf. Zwischen den Tragleisten 3 sind die Behälterzellen 2 entlang der Längsachse L angeordnet und werden durch die beidseitig angeordneten Tragleisten 3 gehalten. Die Behälterzellen 2 weisen jeweils eine Behälterzellenmittelachse M auf, welche senkrecht zur Längsachse L orientiert ist. Die Behälterzellen 2 weisen an ihrer Oberseite eine Einführöffnung 20 auf, durch welche ein zu reinigender Behälter in die Behälterzelle 2 geführt wird. Die Behälterzellen 2 sind untereinander und mit den Tragleisten 3 über Fügebereiche in Form von Fügepunkten 34 fest verbunden.

Im vorliegenden Fall sind die Tragleisten 3 aus Stahlblech hergestellt. Alternativ kann jedoch auch ein anderes Metall beziehungsweise eine andere Metalllegierung oder ein Kunststoff, bevorzugt ein thermoplastischer Kunststoff, zur Herstellung der Tragleisten 3 Anwendung finden. Die Tragleisten 3 weisen jeweils mehrere parallel zur Längsachse L ausgerichtete Biegefalze 37 auf, wodurch sich im Querschnitt der Tragleisten 3 eine Profilform ergibt. Durch die Profilform ist die Stabilität des Behälterzellenträgers 1, insbesondere die Biegesteifigkeiten, gegenüber einem Behälterzellenträger mit einer flachen Tragleiste, beispielsweise aus Flachstahl, deutlich erhöht.

Die Tragleisten 3 weisen ferner einen Fachwerkbereich 38 mit in Richtung der Längsachse L regelmäßig angeordneten Aussparungen 30 zum Ausbilden einer fachwerkartigen Struktur auf, wobei durch die Aussparungen 30 in der Tragleiste 3 Stabsegmente 31 und Knotenpunkte 32 bereitgestellt sind. Jeweils zwei der Stabsegmente 31 treffen sich hierbei in jeweils einem Knotenpunkt 32 des Fachwerkbereichs 38. Durch diese Anordnung der Knotenpunkte 32 und der Stabsegmente 31 können auf die Tragleisten 3 wirkenden Kräfte über die Knotenpunkte 32 direkt von einem Stabsegment 31 zu einem weiteren, denselben Knotenpunkt 32 schneidenden Stabsegment 31 übertragen werden. Zusammen mit dem unteren Rahmen 36 und dem oberen Rahmen 35 der Tragleisten 3 ergibt sich so eine bezogen auf das Eigengewicht der Tragleisten 3 besonders steife Struktur. Im mittleren Bereich der Aussparungen 30 sind parallel zur Längsachse L orientierte Verbindungssegmente 33 angeordnet. Durch diese Verbindungssegmente 33 ist über die gesamte Länge der Tragleisten 3 ein durchgehender Materialabschnitt bereitgestellt, über welchem sich in Richtung der Längsachse L ein Biegefalz 37 erstreckt.

An dem Behälterzellenträger 1 ist stirnseitig eine Anbindungsanordnung 4 angeordnet. Durch diese Anbindungsanordnung 4 kann der Behälterzellenträger 1 in einem Einbauzustand in der Reinigungsmaschine an eine Transportkette angebunden werden. Im vorliegenden Fall ist die Anbindungsanordnung 4 an eine stirnseitig angeordnete Schottwand 6 befestigt, welche die beidseitig angeordneten Tragleisten 3 miteinander verbindet und dem Behälterzellenträger 1 somit zusätzliche Stabilität verleiht. Durch einen Mitnehmer 40 wird hierbei eine Antriebskraft von dem Behälterträger auf Funktionen der Reinigungsmaschine, insbesondere das Drehen der Spritzrohre, übertragen.

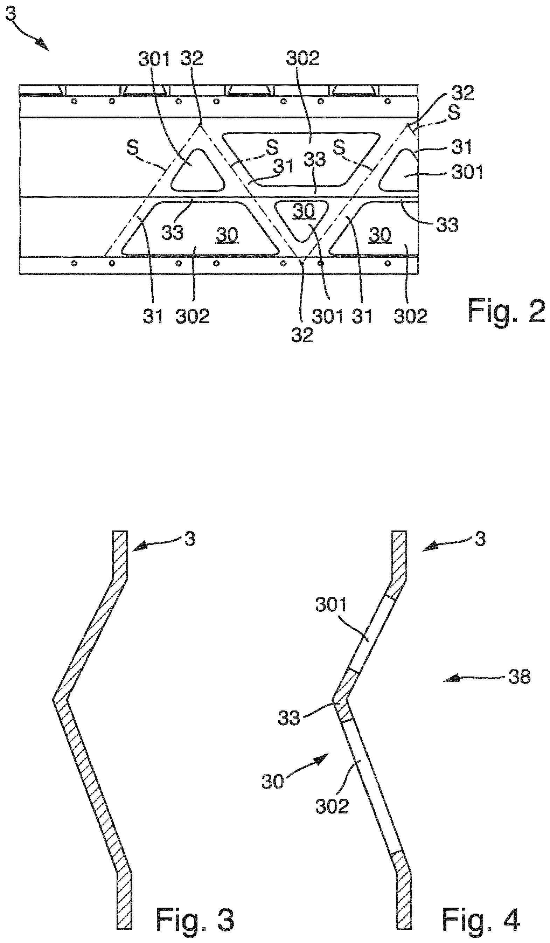

Figur 2 zeigt schematisch eine Tragleiste 3 des Behälterzellenträgers 1 aus Figur 1 in einer Seitenansicht. Die Stabsegmentlängsachsen S zweier Stabsegmente 31 treffen sich jeweils in einem Knotenpunkt 32. Hierdurch ist eine direkte Kraftübertragung von Kräften, deren Vektoren sich in Längsachse L erstrecken, sowohl auch quer dazu möglich.

Die Verbindungssegmente 33, welche in Form von in den Aussparungen 30 an der Tragleiste 3 verbleibende Restblechte ausgebildet sind, teilen die Aussparungen 30 in Aussparungsdreiecke 301 und Aussparungstrapeze 302. Durch den mittels der Verbindungssegmente 33 somit bereitgestellten durchgehenden Materialabschnitt ist die Profilform der Tragleiste 3 zusätzlich stabilisiert.

Die Stabsegmentmittelachsen S schließen einen Winkel mit der Längsachse L ein. Hierdurch können sowohl Kräfte in Richtung der Längsachse L, als auch quer dazu über die Stabsegmente übertragen werden.

Zur Verminderung von Kerbwirkungen, sowie zur Verbesserung des Kraftflusses sind die Ecken der Aussparungen 30 abgerundet.

In Figur 3 ist schematisch ein Querschnitt durch die Trageleiste 3 des Behälterzellenträgers 1 aus Figur 2 senkrecht zur Längsachse L in einem Vollmaterialbereich entlang der Schnittlinie X-X aus Figur 2 gezeigt. Deutlich zu erkennen ist die zweidimensionale Ausprägung der Profilform der Tragleiste 3.

Figur 4 zeigt schematisch einen Querschnitt durch die Trageleiste 3 des Behälterzellenträgers 1 aus Figur 2 senkrecht zur Längsachse L in einem Fachwerkbereich 38 entlang der Schnittlinie Y-Y aus Figur 2. Im Vergleich zum Vollquerschnitt aus Figur 3 zweist der Querschnitt aufgrund der durch das Verbindungssegment 33 in das Aussparungsdreieck 301 und das Aussparungstrapez 302 unterteilten Aussparung 30 eine deutlich reduzierte Fläche auf.

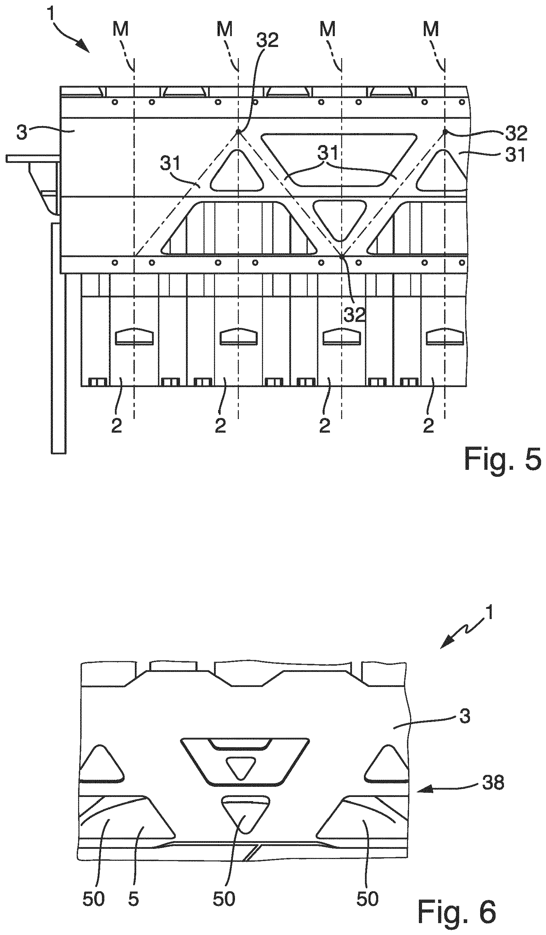

Figur 5 zeigt schematisch eine Seitenansicht des Behälterzellenträgers 1 aus Figur 1. In dieser Ausführungsform liegt jeweils ein Knotenpunkt 32 über einer Behälterzellenmittelachse M. Durch diese Anordnung ist eine besonders stabile Struktur des Behälterzellenträgers 1 bereitgestellt. Die Projektion des Kraftvektors der durch die Behälterzellen 2 in die Tragleisten 3 eingeleiteten Kräfte liegt hierdurch in den Knotenpunkten 32, sodass keine oder in nur sehr geringem Maße Momente auf die Knotenpunkte 32 wirken und die Stabsegmente 31 lediglich Zug- und Druckkräfte übertragen.

Figur 6 zeigt schematisch eine perspektivische Teilansicht eines Behälterzellenträgers in einer alternativen Ausführungsform.

Hierbei sind die Tragleisten 3 über einen an der Oberseite angeordneten Verbindungsbereich 5 als einstückiges U-Profil aus einem gestanzten Biegeblech hergestellt. Der Verbindungsbereich und die Tragleisten sind somit Teilbereiche des umgeformten einstückigen Biegeblechs. Die Behälterzellen werden bei einem Zusammenbau des Behälterzellenträgers 1 in die runden Öffnungen 50 der Verbindungsplatte gesteckt. Die Tragleisten 3 weisen wiederum einen Fachwerkbereich 38 analog zu jenem des Behälterzellenträgers 1 aus Figur 1 bis 5 auf.

Soweit anwendbar können alle einzelnen Merkmale, die in den einzelnen Ausführungsbeispielen dargestellt sind, miteinander kombiniert und/oder ausgetauscht werden, ohne den Bereich der Erfindung zu verlassen.

### Bezugszeichenliste

- 1: Behälterzellenträger
- 2: Behälterzelle
- 20: Einführöffnung
- 3: Tragleiste
- 30: Aussparung
- 301: Aussparungsdreieck
- 302: Aussparungstrapez
- 31: Stabsegment
- 32: Knotenpunkt
- 33: Verbindungssegment
- 34: Fügepunkt
- 35: Oberer Rahmen
- 36: Unterer Rahmen
- 37: Biegefalz
- 38: Fachwerkbereich
- 4: Anbindungsanordnung
- 40: Mitnehmer
- 5: Verbindungsbereich
- 50: Öffnung
- 6: Schottwand

- A: Abstand
- B: Behälterzellenbreite
- L: Längsachse
- M: Behälterzellenmittelachse
- S: Stabsegmentlängsachse
- T: Transportrichtung

- X-X: Schnittlinie
- Y-Y: Schnittlinie

## Patentansprüche

1. Behälterzellenträger (1) zum Tragen mindestens einer Behälterzelle (2) zur Aufnahme eines zu reinigenden Behälters in einer Reinigungsmaschine, bevorzugt in einer Reinigungsmaschine einer Getränkeabfüllanlage, umfassend mindestens eine sich in einer Längsachse (L) erstreckende Tragleiste (3) zum Halten der mindestens einen Behälterzelle (2) zum Transport durch die Reinigungsmaschine,
die mindestens eine Tragleiste (3) einen Fachwerkbereich (38) mit in Richtung der Längsachse (L) regelmäßig angeordneten Aussparungen (30) zum Ausbilden einer fachwerkartigen Struktur aufweist, wobei durch die Aussparungen (30) in der Tragleiste (3) Stabsegmente (31) und Knotenpunkte (32) bereitgestellt sind und wobei sich jeweils mindestens zwei der Stabsegmente (31) in jeweils einem Knotenpunkt (32) des Fachwerkbereichs (38) treffen
**dadurch gekennzeichnet, dass**
mindestens ein Knotenpunkt (32) des Fachwerkbereichs (38) in Richtung der Längsachse (L) gesehen auf Höhe einer senkrecht zur Längsachse (L) orientierten Behälterzellenmittelachse (M) mindestens einer Behälterzelle (2) angeordnet ist.

2. Behälterzellenträger (1) gemäß einem der vorstehenden Ansprüche, **dadurch**
**gekennzeichnet, dass** jeweils genau ein Knotenpunkt (32) des Fachwerkbereichs (38) in Richtung der Längsachse (L) gesehen auf Höhe der Behälterzellenmittelachse (M) jeweils einer Behälterzelle (2) angeordnet ist.

3. Behälterzellenträger (1) gemäß einem der vorstehenden Ansprüche, **dadurch**
**gekennzeichnet, dass** die Stabsegmente (31) mit ihrer Stabsegmentlängsachse (S) mit der Längsachse (L) einen Winkel, bevorzugt in einem Winkelbereich von größer 0° und kleiner 90°, einschließen.

4. Behälterzellenträger (1) gemäß einem der vorstehenden Ansprüche, **dadurch**
**gekennzeichnet, dass** die Gesamtfläche der Aussparungen (30) höchstens 50 % der Gesamtfläche der Tragleiste (3) beträgt.

5. Behälterzellenträger (1) gemäß einem der vorstehenden Ansprüche, **dadurch**
**gekennzeichnet, dass** die Behälterzellen (2) mit einem Abstand (A) zwischen den Behälterzellenmittelachsen (M) zweier benachbarter Behälterzellen (2) angeordnet sind, wobei die Behälterzellen (2) eine Behälterzellenbreite (B) aufweisen und wobei der Abstand (A) bevorzugt der Behälterzellenbreite (B) entspricht.

6. Behälterzellenträger (1) gemäß einem der vorstehenden Ansprüche, **dadurch**
**gekennzeichnet, dass** mindestens eine Aussparung (30) als Dreieck bereitgestellt ist, bevorzugt sind alle Aussparungen (30) als Dreiecke bereitgestellt.

7. Behälterzellenträger (1) gemäß einem der vorstehenden Ansprüche, **dadurch**
**gekennzeichnet, dass** mindestens eine Ecke mindestens einer Aussparung (30) abgerundet ist, bevorzugt sind alle Ecken der Aussparung (30), besonders bevorzugt alle Ecken aller Aussparungen (30), abgerundet bereitgestellt.

8. Behälterzellenträger (1) gemäß einem der vorstehenden Ansprüche, **dadurch**
**gekennzeichnet, dass** in dem Fachwerkbereich (38) mindestens ein Verbindungssegment (33) zum Versteifen des Fachwerkbereichs (38) zwischen den Stabsegmenten (31), bevorzugt parallel zur Längsachse (L) orientiert, angeordnet ist und/oder zwischen den Tragleisten (3) senkrecht zur Längsachse (L) mindestens eine Schottwand (6) zum Versteifen des Behälterzellenträgers (1) angeordnet ist, bevorzugt wenn die mindestens eine Behälterzelle Kunststoff aufweist beziehungsweise aus Kunststoff besteht.

9. Behälterzellenträger (1) gemäß einem der vorstehenden Ansprüche, **dadurch**
**gekennzeichnet, dass** die mindestens eine Tragleiste (3) als Profilleiste bereitgestellt.

10. Behälterzellenträger (1) gemäß einem der vorstehenden Ansprüche, **dadurch**
**gekennzeichnet, dass** ein Verbindungsbereich (5) zum Verbinden mindestens zweier Tragleisten (3) bereitgestellt ist, wobei die Tragleisten (3) beidseitig an dem Verbindungsbereich (5) angeordnet sind und wobei die Tragleisten (3) und der Verbindungsbereich (5) bevorzugt als einstückiges U-Profil bereitgestellt sind, wobei der Verbindungsbereich (5) bevorzugt seitlich, oberhalb und/oder unterhalb der Tragleisten (3) angeordnet ist.

11. Behälterzellenträger (1) gemäß einem der vorsehenden Ansprüche, **dadurch**
**gekennzeichnet, dass** mindestens eine Anbindungsanordnung (4) zum Anbinden an eine Transportkette der Reinigungsmaschine, bevorzugt stirnseitig, angeordnet ist.

12. Behälterzellenträger (1) gemäß einem der vorstehenden Ansprüche, **dadurch**
**gekennzeichnet, dass** die mindestens eine Behälterzelle (2) eingesteckt ist.

13. Behälterzellenträger (1), gemäß einem der vorstehenden Ansprüche, **dadurch**
**gekennzeichnet, dass** die mindestens eine Behälterzelle (2) mittels mindestens eines Fügebereichs, bevorzugt mittels Kleben, Schweißen, Verschrauben, Nieten und/oder Schmelzen, an der mindestens einen Tragleiste (3) gehalten ist.

14. Behälterzellenträger (1) gemäß einem der vorstehenden Ansprüche, **dadurch**
**gekennzeichnet, dass** die Aussparungen (30) durch Heraustrennen, bevorzugt Stanzen und/oder ein Beschnittverfahren wie Laserstrahlschneiden oder Wasserstrahlschneiden, aus einem Vollmaterial der mindestens einen Tragleiste (3) bereitgestellt sind, wobei die mindestens eine Tragleiste (3) aus einem Kunststoff und/oder einem Metall oder einer Metalllegierung, bevorzugt Stahl, besonders bevorzugt als Biegeblech, Stanzblech, Spritzgußteil und/oder Extrusionsprofil, bereitgestellt ist.

15. Reinigungsmaschine zum Reinigen von Behältern, bevorzugt in einer Getränkeabfüllanlage, umfassend eine Transportkette mit einer Transportrichtung (T) und mindestens eine Reinigungseinrichtung zum Reinigen der Behälter,
**dadurch gekennzeichnet, dass**
eine Vielzahl an Behälterzellenträgern (1) gemäß einem der vorstehenden Ansprüche entlang der Transportkette der Reinigungsmaschine angeordnet ist, wobei die Behälterzellenträger (1) hinsichtlich ihrer Längsachse (L) quer zu der Transportrichtung (T) angeordnet sind.

## Claims

1. Container cell carrier (1) for carrying at least one container cell (2) for receiving a container to be cleaned in a cleaning machine, preferably in a cleaning machine of a drinks filling plant, comprising at least one support bar (3) extending in a longitudinal axis (L) for holding the at least one container cell (2) for transport through the cleaning machine; the at least one support bar (3) has a framework region (38) having recesses (30) arranged regularly in the direction of the longitudinal axis (L) for forming a framework-type structure, wherein rod segments (31) and nodal points (32) are provided by the recesses (30) in the support bar (3) and wherein at least two of the rod segments (31) respectively meet at one nodal point (32) of the framework region (38) respectively,
**characterised in that,**
seen in the direction of the longitudinal axis (L) at least one nodal point (32) of the framework region (38) is arranged at the height of a container cell central axis (M) of at least one container cell (2), said container cell central axis oriented perpendicularly to the longitudinal axis (L).

2. Container cell carrier (1) according to any one of the preceding claims,
**characterised in that,** seen in the direction of the longitudinal axis (L), exactly one nodal point (32) of the framework region (38) respectively is arranged at the height of the container cell central axis (M) of a container cell (2) respectively.

3. Container cell carrier (1) according to any one of the preceding claims,
**characterised in that** the rod segments (31) enclose an angle with their rod segment longitudinal axis (S) with the longitudinal axis (L), preferably in an angular range of greater than 0° and less than 90°.

4. Container cell carrier (1) according to any one of the preceding claims,
**characterised in that** the total area of the recesses (30) amounts to at most 50% of the total area of the support bar (3).

5. Container cell carrier (1) according to any one of the preceding claims,
**characterised in that** the container cells (2) are arranged with a distance (A) between the container cell central axes (M) of two adjacent container cells (2), wherein the container cells (2) have a container cell width (B) and wherein the distance (A) preferably corresponds to the container cell width (B).

6. Container cell carrier (1) according to any one of the preceding claims,
**characterised in that** at least one recess (30) is provided as a triangle, preferably all recesses (30) are provided as triangles.

7. Container cell carrier (1) according to any one of the preceding claims,
**characterised in that** at least one corner of at least one recess (30) is rounded, preferably all corners of the recess (30), particularly preferably all corners of all recesses (30) are provided in a rounded state.

8. Container cell carrier (1) according to any one of the preceding claims,
**characterised in that,** in the framework region (38), at least one connection segment (33) for stiffening the framework region (38) is arranged between the rod segments (31), preferably oriented in parallel to the longitudinal axis (L), and/or at least one partition wall (6) for stiffening the container cell carrier (1) is arranged between the support bars (3) perpendicularly to the longitudinal axis (L), preferably if the at least one container cell comprises plastic or consists of plastic.

9. Container cell carrier (1) according to any one of the preceding claims,
**characterised in that** the at least one support bar (3) is provided as a profiled bar.

10. Container cell carrier (1) according to any one of the preceding claims,
**characterised in that** a connection region (5) for connecting at least two support bars (3) is provided, wherein the support bars (3) are arranged on both sides of the connection region (5) and wherein the support bars (3) and the connection region (5) are preferably provided as a single-piece U-profile, wherein the connection region (5) is preferably arranged to the side, above and/or below the support bars (3).

11. Container cell carrier (1) according to any one of the preceding claims,
**characterised in that** at least one linking arrangement (4) is arranged for linking to a transport chain of the cleaning machine, preferably on the front side.

12. Container cell carrier (1) according to any one of the preceding claims,
**characterised in that** the at least one container cell (2) is inserted.

13. Container cell carrier (1) according to any one of the preceding claims,
**characterised in that** at least one container cell (2) is held on the at least one support bar (3) by means of at least one joining region, preferably by means of adhesive bonding, welding, screwing together, riveting and/or smelting.

14. Container cell carrier (1) according to any one of the preceding claims,
**characterised in that** the recesses (30) are provided by separating, preferably by punching and/or a cutting method such as laser beam cutting or water jet cutting, from a solid material of the at least one support bar (3), wherein the at least one support bar (3) is provided to be made from a plastic and/or a metal or a metal alloy, preferably steel, particularly preferably as a bending plate, a punched plate, an injection moulded part and/or an extruded profile.

15. Cleaning machine for cleaning containers, preferably in a drinks filling plant, comprising a transport chain having a transport direction (T) and at least one cleaning device for cleaning the containers,
**characterised in that**
a plurality of container cell carriers (1) according to any one of the preceding claims is arranged along the transport chain of the cleaning machine, wherein the container cell carriers (1) are arranged transversely to the transport direction (T) with regard to their longitudinal axis (L).

## Revendications

1. Support de cellule de récipient (1) pour supporter au moins une cellule de récipient (2) afin de recevoir un récipient à nettoyer dans une machine de nettoyage, de préférence dans une machine de nettoyage d'une installation d'embouteillage de boissons, comprenant au moins une lisse de suspension (3) s'étendant selon un axe longitudinal (L) pour maintenir la au moins une cellule de récipient (2) pour le transport à travers la machine de nettoyage,
la au moins une lisse de suspension (3) présente une zone de treillis (38) avec des évidements (30) ménagés de manière régulière dans la direction de l'axe longitudinal (L) afin de former une structure en treillis, dans lequel les évidements (30) de la lisse de suspension (3) forment des segments de barre (31) et des points de jonction (32) et dans lequel respectivement au moins deux des segments de barre (31) se rencontrent dans respectivement un point de jonction (32) de la zone en treillis (38),
**caractérisé en ce que** :
au moins un point de jonction (32) de la zone en treillis (38) est agencé, en observant dans la direction de l'axe longitudinal (L), à hauteur d'un axe central de cellule de récipient (M), orienté perpendiculairement à l'axe longitudinal (L), d'au moins une cellule de récipient (2).

2. Support de cellule de récipient (1) selon l'une quelconque des revendications précédentes, **caractérisé en ce que** respectivement exactement un point de jonction (32) de la zone en treillis (38) est agencé, en observant dans la direction de l'axe longitudinal (L), à hauteur de l'axe central de cellule de récipient (M) respectivement d'une cellule de récipient (2).

3. Support de cellule de récipient (1) selon l'une quelconque des revendications précédentes, **caractérisé en ce que** les segments de barre (31) avec leur axe longitudinal de segment de barre (S) font avec l'axe longitudinal (L) un angle, de préférence dans une zone angulaire de plus de 0° et de moins de 90°.

4. Support de cellule de récipient (1) selon l'une quelconque des revendications précédentes, **caractérisé en ce que** la surface totale des évidements (30) atteint au maximum 50 % de la surface totale de la lisse de suspension (3).

5. Support de cellule de récipient (1) selon l'une quelconque des revendications précédentes, **caractérisé en ce que** les cellules de récipients (2) sont agencées avec une distance (A) entre les axes centraux (M) de deux cellules de récipients voisines (2), dans lequel les cellules de récipients (2) présentent une largeur de cellules de récipients (B) et dans lequel la distance (A) correspond de préférence à la largeur de cellules de récipients (B).

6. Support de cellule de récipient (1) selon l'une quelconque des revendications précédentes, **caractérisé en ce qu'**au moins un évidement (30) se présente sous la forme d'un triangle, de préférence tous les évidements (30) se présentent sous la forme de triangles.

7. Support de cellule de récipient (1) selon l'une quelconque des revendications précédentes, **caractérisé en ce qu'**au moins un angle d'au moins un évidement (30) est arrondi, de préférence tous les angles de l'évidement (30), tout particulièrement tous les angles de tous les évidements (30) se présentent sous forme arrondie.

8. Support de cellule de récipient (1) selon l'une quelconque des revendications précédentes, **caractérisé en ce que**, dans la zone en treillis (38), au moins un segment de liaison (33) est agencé pour renforcer la zone en treillis (38) entre les segments de barre (31), de préférence avec une orientation parallèle à l'axe longitudinal (L) et/ou au moins une cloison étanche (6) pour renforcer le support de cellule de récipient (1) est agencée entre les lisses de suspension (3) perpendiculairement à l'axe longitudinal (L), de préférence lorsque la au moins une cellule de récipient comporte une matière plastique ou est constituée d'une matière plastique.

9. Support de cellule de récipient (1) selon l'une quelconque des revendications précédentes, **caractérisé en ce que** la au moins une lisse de suspension (3) se présente sous la forme d'une lisse profilée.

10. Support de cellule de récipient (1) selon l'une quelconque des revendications précédentes, **caractérisé en ce qu'**une zone de liaison (5) est mise en oeuvre pour relier au moins deux lisses de suspension (3), dans lequel les lisses de suspension (3) sont agencées des deux côtés de la zone de liaison (5) et dans lequel les lisses de suspension (3) et la zone de liaison (5) se présentent de préférence sous la forme d'un profil en U d'un seul tenant, dans lequel la zone de liaison (5) est agencée de préférence latéralement, au-dessus et/ou au-dessous des lisses de suspension (3).

11. Support de cellule de récipient (1) selon l'une quelconque des revendications précédentes, **caractérisé en ce qu'**au moins un agencement de rattachement (4) est agencé pour se rattacher à une chaîne de transport de la machine de nettoyage, de préférence côté frontal.

12. Support de cellule de récipient (1) selon l'une quelconque des revendications précédentes, **caractérisé en ce que** la au moins une cellule de récipient (2) est enfichée.

13. Support de cellule de récipient (1) selon l'une quelconque des revendications précédentes, **caractérisé en ce que** la au moins une cellule de récipient (2) est maintenue sur la au moins une lisse de suspension (3) au moyen d'au moins une zone d'assemblage, de préférence par collage, soudage, vissage, rivetage et/ou fusion.

14. Support de cellule de récipient (1) selon l'une quelconque des revendications précédentes, **caractérisé en ce que** les évidements (30) sont ménagés par refoulement, de préférence par estampage et/ou par un procédé de coupe tel qu'une coupe par rayon laser ou une coupe par jets d'eau, sur un matériau plein de la au moins une lisse de suspension (3), dans lequel la au moins une lisse de suspension (3) est élaborée à partir d'une matière plastique et/ou d'un métal ou d'un alliage métallique, de préférence de l'acier, tout particulièrement en partant d'une tôle de pliage, d'une tôle d'estampage, d'une pièce moulée par injection et/ou d'un profilé d'extrusion.

15. Machine de nettoyage pour nettoyer des récipients, de préférence dans une installation d'embouteillage de boissons, comprenant une chaîne de transport avec une direction de transport (T) et au moins un dispositif de nettoyage pour nettoyer les récipients, **caractérisée en ce que** :
une pluralité de supports de cellules de récipients (1) selon l'une quelconque des revendications précédentes est agencée le long de la chaîne de transport de la machine de nettoyage, dans laquelle les supports de cellules de récipients (1) sont agencés avec leur axe longitudinal (L) transversal à la direction de transport (T).
